Europäisches Patentamt

European Patent Office

Office européen des brevets

(11) Veröffentlichungsnummer: **0 428 061 A1**

## (12) EUROPÄISCHE PATENTANMELDUNG

(21) Anmeldenummer: 90121350.4

(22) Anmeldetag: 08.11.90

(51) Int. Cl.5: **C12N 9/22, C12N 1/20, //(C12N1/20,C12R1:265)**

---

Le (Les) microorganisme(s) a (ont) été déposé(s) auprès Deutsche Sammlung von Mikroorganismen und Zellkulturen sous le numéro 20499.

(30) Priorität: **16.11.89 DE 3938144**

(43) Veröffentlichungstag der Anmeldung:
**22.05.91 Patentblatt 91/21**

(84) Benannte Vertragsstaaten:
**AT BE CH DE DK ES FR GB GR IT LI LU NL SE**

(71) Anmelder: **BOEHRINGER MANNHEIM GMBH**
**Sandhofer Strasse 116**
**W-6800 Mannheim 31(DE)**

(72) Erfinder: **Kaluza, Klaus, Dr. rer. nat.**
**Hochfeldanger 3**
**W-8173 Bad Heilbrunn(DE)**
Erfinder: **Hoeltke, Hans Joachim, Dr. rer. nat.**
**Kirchenstrasse 1/I**
**W-8132 Tutzing(DE)**
Erfinder: **Jarsch, Michael, Dr. rer. nat.**
**Unterkarpfsee 11**
**W-8173 Bad Heilbrunn(DE)**
Erfinder: **Schmitz-Agheguian, Gudrun, Dr. rer. nat.**
**Wettersteinstrasse 3**
**W-8139 Bernried(DE)**
Erfinder: **Kessler, Christoph, Dr. rer. nat.**
**Schlossbergweg 11**
**W-8021 Dorfen(DE)**

---

(54) **Typ II-Restriktionsendonuklease McrI.**

(57) Die neue TypII-Restriktionsendonuklease McrI besitzt folgende Erkennungssequenz, spaltet bevorzugt an der durch die Markierung definierten Spaltstelle:

$$5'-CG \quad \genfrac{}{}{0pt}{}{GC}{AT} \Big| CG-3'$$

$$3'-GC \Big| \genfrac{}{}{0pt}{}{CG}{TA} \quad GC-5'$$

Sie ist vorzugsweise aus Mikroorganismen der Gattung Micrococcus erhältlich.

# TYP II-RESTRIKTIONSENDONUKLEASE MCRI

Die Erfindung betrifft die neue Typ II-Restriktionsendonuklease Mcrl, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen, die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Typ-II-Restriktionsendonuklease mit der Erkennungssequenz und der durch die Markierung definierten Spaltstelle

$$5'-CG \; \frac{GC}{AT} \Big| CG-3'$$
$$3'-GC \Big| \frac{CG}{TA} \; GC-5'$$

Die erfindungsgemäße neue Restriktionsendonuklease, die im folgenden als Mcrl bezeichnet wird, hat ein Temperaturoptimum bei 37° C. Das Enzym besitzt eine gute Aktivität zwischen pH 7,4 und pH 8,3 in 50 mmol/l Tris/HCl-Puffer mit 1,0 mmol/l DTE (Dithioerythritol) 10 mmol/l Mg-acetat. Das pH-Optimum liegt bei pH 7,8. Ein Enzym, das isoschizomer zu Mcrl ist, ist nicht bekannt.

Die Erkennungssequenz läßt sich durch vollständigen Verdau des Virus Adeno 2, der Phagen Lambda, phiX174 und dem Phagenderivaten M13mp8 und den Plasmiden pBR322 und pBR328 bestätigen. Diese DNA-Moleküle werden mit Mcrl behandelt.

Tabelle 1 zeigt einen Vergleich der experimentell beobachteten Schnittstellenspezifität mit einer computerermittelten Schnittstellenspezifität für ein Enzym, welches folgende Sequenz erkennt:

$$5'-CG\frac{GC}{AT}CG-3'$$

| Tabelle I | | | | | | | | | | | |
|---|---|---|---|---|---|---|---|---|---|---|---|
| DNA | Anzahl der experimentell bestimmten Schnittstellen | Anzahl der durch Computeranalyse bestimmte Schnittstellen | Experimentell bestimmte Fragmentlängen (Basenpaare) | | | Durch Computeranalyse bestimmte Fragmentlängen (Basenpaare) | | | Spaltpositionen ermittelt durch Computeranalyse (bei Basenpaar) | | |
| SV40 | | 0 | | | | | 0 | | | | |
| M13mp8 | | 4 | 100 | 2100 | 2450 | 116 | 2151 | | 1421 | 3878 | |
| | | | 2500 | | | 2457 | 2505 | | 6383 | 6499 | |
| phiX174 | | 1 | | 5380 | | | 5386 | | 4601 | | |
| pBR322 | | 7 | 150 | 280 | 370 | .149 | 286 | 367 | 286 | 653 | |
| | | | 420 | 770 | 920 | 424 | 765 | 923 | 2388 | 2812 | 3735 |
| | | | 1500 | | | 1449 | | | 3884 | | |
| pBR328 | | 8 | 150 | 280 | 370 | 149 | 286 | 367 | 286 | 653 | |
| | | | 400 | 560 | 780 | 395 | 560 | 784 | 939 | 1723 | 2646 |
| | | | 920 | 1440 | | 923 | 1443 | | 2795 | 4238 | 4798 |

EP 0 428 061 A1

Die Spaltposition innerhalb der Erkennungssequenz des Enzyms läßt sich an einem M13-Derivat, das im Abstand von ca. 30 - 200 Basen zur Bindungsstelle des universalen Sequenzierprimers (Messing, J. et al. (1981) Nucl. Acids Res. 9, 309 - 321) diese Erkennungssequenz trägt, bestimmen. An einzelsträngiger DNS des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74, 560 - 564, Messing, J. et al. (1981) Nucl. Acids Res. 9, 309 -321).

Parallel dazu wird der Sequenzierprimer mit T4-Polynukleotid-Kinase und $[\gamma\text{-}^{32}\text{P}]$ATP am 5′-Ende radioaktiv markiert. Nach Anhybridisieren dieses 5′-endmarkierten Sequenzierprimers an die einzelsträngige M13-DNS wird in einer Auffüllreaktion mit DNS-Polymerase I (Klenow-Enzym) und einer Desoxynukleotidtriphosphatmischung aus dATP, dCTP, dGTP und dTTP eine partiell doppelsträngige DNS hergestellt. Diese DNS, deren neusynthetisierter Strang am 5′-Ende radioaktiv markiert ist, wird nun mit der Restriktionsendonuklease McrI gespalten. Die Hälfte des Spaltansatzes wird zusätzlich noch mit T4-DNS-Polymerase in Gegenwart einer Mischung aller vier Desoxynukleotidtriphosphate behandelt, um glatte DNS-Enden zu erhalten.

Die Analyse der Reaktionsprodukte erfolgt durch Elektrophorese auf Sequenziergelen (8 mol/l Harnstoff, 5% Polyacrylamid) und anschließender Autoradiographie. Die Interpretation der Ergebnisse wird nach Brown, N.L. und Smith, M. (Methods in Enzymology 65 (1980) 391 -401) durchgeführt. Durch Vergleich der Laufstrecken der radioaktiv markierten Fragmente mit der Sequenzleiter wird die Lage der Spaltstelle bestimmt. Die zusätzlich mit T4 DNS Polymerase behandelten Proben zeigen im Vergleich zu der lediglich McrI-gespaltenen Probe eine um 2 bp verlängerte Laufstrecke der Banden. Damit ist gezeigt, daß McrI 3′ überhängende DNS-Enden erzeugt. Die Spaltung von McrI erfolgt innerhalb der Erkennungssequenz daher mit folgender Spezifität:

$$5'\text{-CG}\begin{array}{|c}\text{GC}\\\text{AT}\end{array}\text{CG-3}'$$
$$3'\text{-GC}\begin{array}{c|}\text{CG}\\\text{TA}\end{array}\text{GC-5}'$$

Die experimentell bestimmte Anzahl der Schnittstellen ist identisch mit der Anzahl der Schnittstellen, die durch Computeranalyse mit den verschiedenen DNAs für die Sequenz

$$5'\text{-CG}^{\text{GC}}_{\text{AT}}\text{CG-3}'$$

erhalten wurde (Tabelle I). Zusätzlich wurden diese Daten noch mit den Tabellen aus Gene 10 (1980) 357 -370, verglichen.

Vorzugsweise wird McrI gewonnen, indem man Mikroorganismen der Gattung Micrococcus züchtet und das Enzym aus den Zellen gewinnt. Vorzugsweise wird Micrococcus cryophilus DSM 20429 verwendet.

Der Mikroorganismus ist bei der Deutschen Sammlung von Mikroorganismen (DSM), Mascheroder Weg 1 b, 3300 Braunschweig, BRD, hinterlegt und trägt die Hinterlegungsnummer DSM 20429.

Die für die Gewinnung des Enzyms verwendeten Microorganismen wachsen aerob in Merck Standard I Medium.

Die optimalen Wachstumsbedingungen sind bei 18°C, pH 7,2 - 7,8. Die Verdoppelungszeit beträgt etwa 2 Stunden.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen einem Überdruck von 5 bar ausgesetzt. Die hierbei entstandene Zellmasse wird in Tris-HCl Puffer, pH 8,0, welcher Protease-Inhibitoren enthält, resuspendiert. Anschliessend werden die Zellen über eine French-Presse aufgechlossen. Die weitere Reinigung des Überstands wird vorzugsweise über Afinitätschromatographie und Ionentauscherchromatograpgie durchgeführt. Als Material für die Affinitätschromatographie geeignet ist beispielsweise Heparin-Sepharose CL-6B (Pharmacia).

Als Anionentauscher geeignet ist das unter dem Namen Q-Sepharose (Pharmacia) erhältliche Produkt. Aber auch andere Chromatographiematerialien, die dem Fachmann bekannt sind, sind geeignet.

Die folgenden Beispiele erläutern die Erfindung weiter.

Beispiel 1

Micrococcus cryophilus DSM 20429 wird bei 18°C 25 Stunden gezüchtet und in der spätlogarithmischen Phase geerntet. Als Kulturmedium wird Merck Standard I-Medium verwendet.

Die Zellpaste (30g Naßgewicht) wird in 2,4 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch² aufgeschlossen und der Niederschlag abgetrennt. Der Überstand wird mit $NH_4Cl$ versetzt (Endkonzentration 0,6 mol/l). Mit einer Polymin-Fällung werden die Nucleinsäuren entfernt. Anschließend wird der abzentrifugierte Überstand mit 55% Ammoniumsulfat behandelt. Der Niederschlag wird in Puffer B (40 mmol/l Tris-HCl, pfl 8,0; 0,1 mmol/l EDTA; 7 mmol/l 2-Mercaptoethanol, 10% (w/v) Glycerin) aufgenommen und über eine Q-Sepharose-Säule fraktioniert. Zur Elution wird ein Gradient von 0 - 0,5 mol/l NaCl verwendet. Mcrl wird in den Fraktionen zwischen 0,2 - 0,3 mol/l NaCl gefunden. Die aktiven Fraktionen werden auf einer S-Sepharose-Säule fraktioniert. Zur Elution wird ein Gradient von 0 - 1 mol/l NaCl verwendet. Die aktiven Fraktionen, zwischen 0,4 - 0,5 mol/l NaCl, werden gegen Puffer B equilibriert und auf eine Heparin-Sepharose-Säule aufgegeben. Zur Elution wird ein Gradient von 0 - 1 mol/l NaCl in Puffer B verwendet. Mcrl wird in den Fraktionen zwischen 0,4 und 0,6 mol/l NaCl gefunden.

Die aktiven Fraktionen werden zusammengegeben und gegen Lagerpuffer (20 mmol/l Tris-HCl, pH 8,0, 10 mmol/l 2-Mercaptoethanol, 100 mmol/l NaCl, 0,1 mmol/l EDTA und 50% (v/v) Glycerin) dialysiert.

Beispiel 2

Bestimmung der Aktivität

Definition der Enzymeinheiten:

1 U Mcrl spaltet 1 μg Lambda-DNA innerhalb 1 Stunde bei 37°C in 25 μl Endvolumen.

Zu einer Mischung von 2,5 μl Inkubationspuffer (500 mmol/l Tris-HCl, pH 7,5, 37°C, 100 mmol/l Magnesiumacetat, 1 mol/l NaCl und 10 mmol/l DTE) werden 17,9 μl Wasser und 3,6 μl Lambda DNA (optische Dichte: 5,6 OD/ml) sowie 1 μl Mcrl-Lösung (1 U/μl) zugegeben.

Die Lösung wird eine Stunde bei 37°C inkubiert, auf Eis gekühlt und mit 5 μl eines Abstopp-Reagenses, bestehend aus 7 mmol/l Harnstoff, 20 % (w/v) Saccharose, 60 mmol/l EDTA und 0,01 % (w/v) Bromphenolblau versetzt.

Anschließend wird eine Trennung durch Elektrophorese in 1 % Agarose-Gelen für 3 - 4 Stunden bei 100 V durchgeführt. Die erhaltenen Banden werden über Vergleich mit einem DNA-Längenstandard identifiziert.

**Ansprüche**

1. Typ II-Restriktionsendonuklease mit der Erkennungs sequenz und der durch die Markierung charakterisierten Schnittstelle

$$5'\text{-CG} \begin{smallmatrix}GC\\AT\end{smallmatrix}\Big| CG\text{-}3'$$

$$3'\text{-GC} \Big| \begin{smallmatrix}CG\\TA\end{smallmatrix} GC\text{-}5'$$

2. Typ II-Restriktionsendonuklease nach Anspruch 1 dadurch gekennzeichnet, daß sie aus Mikroorganismen der Gattung Micrococcus cryophilus erhältlich ist.

3. Restriktionsendonuklease nach Anspruch 1 oder 2 dadurch gekennzeichnet, daß sie aus Micrococcus cryophilus DSM 20429 erhältlich ist.

4. Restriktionsendonuklease nach den Ansprüchen 1 bis 3, gekennzeichnet durch ein Temperatur-Optimum bei 37°C und ein pH-Optimum zwischen 7,5 und 8,0.

5. Verfahren zur Gewinnung einer TypII-Restriktionsendonuklease mit der Erkennungssequenz und der durch die Markierung charakterisierten Spaltstelle

$$5'\text{-CG} \begin{smallmatrix}GC\\AT\end{smallmatrix}\Big| CG\text{-}3'$$

$$3'\text{-GC} \Big| \begin{smallmatrix}CG\\TA\end{smallmatrix} GC\text{-}5'$$

dadurch gekennzeichnet, daß man Mikroorganismen der Gattung Micrococcus züchtet und das Enzym aus den Zellen gewinnt.

6. Verfahren nach Anspruch 5, dadurch gekennzeichnet, daß man Micrococcus cryophilus DSM 20429 züchtet.

7. Verfahren nach den Ansprüchen 5 und 6, dadurch gekennzeichnet, daß man die Zellen aufschließt und den Zellüberstand gewinnt.

8. Verfahren nach Anspruch 7, dadurch gekennzeichnet, daß man zur Feinreinigung den Zellüberstand einer Affinitätschchromatographie, einer Anionenaustauschchromatographie und einer Kationenaustauschchromatographie unterwirft.

9. Verfahren nach Anspruch 8, dadurch gekennzeichnet, daß man zur Affinitätschchromatographie trägerfixiertes Heparin verwendet.

10. Verwendung einer TypII-Restriktionsendonuklease mit der Erkennungssequenz und der durch die Markierung charakterisierten Schnittstelle

$$5'\text{-CG} \begin{smallmatrix}GC\\AT\end{smallmatrix}\Big| CG\text{-}3'$$

$$3'\text{-GC} \Big| \begin{smallmatrix}CG\\TA\end{smallmatrix} GC\text{-}5'$$

zur Erkennung und Spaltung der komplementären doppelsträngigen DNA-Sequenz

$$5'\text{-CG}\begin{smallmatrix}GC\\AT\end{smallmatrix}\text{CG-}3'$$

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | FEBS LETTERS. vol. 264, no. 2, 21 Mai 1990, AMSTERDAM NL Seiten 218 - 222; BRENSING-KUPPERS,J. et al: "McrI: a novel class-II restriction endonuclease from Micrococcus cryophilus recognizing 5'-CGRY/CG-3'" * das ganze Dokument * ----- | 1-10 | C12N9/22 C12N1/20 //(C12N1/20, C12R1:265) |

|  |  |  | RECHERCHIERTE SACHGEBIETE (Int. Cl.5 ) |
|---|---|---|---|
|  |  |  | C12N |

Der vorliegende Recherchenbericht wurde für alle Patentansprüche erstellt

| Recherchenort | Abschlußdatum der Recherche | Prüfer |
|---|---|---|
| DEN HAAG | 12 FEBRUAR 1991 | ANDRES S.M. |